# EUROPEAN PATENT APPLICATION

(11) **EP 1 724 239 A1**
(43) Date of publication of application: **22.11.2006**
(21) Application number: 06017131.1
(22) Date of filing: 24.03.2004
(51) Int. Cl.: C01F 11/18, A61K 8/19

(54) **A treatment composition**

(30) Priority: 24.03.2003 WO PCT/EP03/03052; 29.07.2003 IE 20030560
(62) Divisional of application: 04722926.5
(71) Applicant: Exa SA, 1295 Mies (CH)
(72) Inventor: The designation of the inventor has not yet been filed
(74) Representative: Purdy, Hugh Barry

(57) **Abstract**

Use of a particulate erasing agent in a personal wash composition is described. The particulate erasing agent is of the type comprising a plurality of particles, the particles comprising a precipitate or agglomerate of an insoluble alkali metal carbonate, being generally round and having an irregular surface configuration, being dimensioned to roll along a surface, and having an average maximum diameter of between 30 and 1000 microns. Generally, the precipitated alkali metal carbonate is precipitated calcium carbonate. The personal wash composition is selected from the group comprising: soap; shower gel; and body wash. Also described is a personal washing composition comprising the particulate erasing agent.

## Description

### Technical Field

The invention relates to a treatment composition for treating a surface. In particular, the invention relates to a composition for treating teeth and household cleaning surfaces.

### Background

A great many toothpaste compositions have been developed and marketed for several years now.

It is known that toothpaste formulations may contain various components, in particular water, a wetting agent (for example glycerol, sorbitol, xylitol or polyethylene glycol, etc.), a thickener (for example xanthan gum), a source of flouride (usually sodium fluoride or sodium monoflurophosphate (anti-tooth-decay), a colorant, a flavouring, a sweetener, a fragrance, a preserving agent, a surfactant and/or additive, etc.

They generally also contain an abrasive agent which must, by its mechanical action, remove dental plaque while at the same time not subjecting the teeth themselves to unacceptable abrasion.

Among the abrasive agents usually employed, mention may be made of sodium bicarbonates and calcium phosphates, sodium metaphosphates, aluminas and, in recent years, silicas.

However, the agents of the prior art, in particular silica and alumina abrasive agents in toothpaste compositions, are not always of desirable refractive index or porosity.

It is an object of the invention to overcome at least some of the above disadvantages.

### Statements of Invention

According to the invention, there is provided a treatment composition which comprises a particulate erasing agent, the particles of the erasing agent being dimensioned to roll along a surface. In one embodiment, the treatment composition is a personal care treatment composition, such as, for example, a dental care treatment composition. Other types of personal care treatments include skin exfoliation and personal washing.

In this specification, the term "particulate erasing agent" should be understood as referring to a multiplicity of relatively soft particles which are dimensioned to be rolled along a surface and which, during such a rolling action, pick up debris, stains, plaque, tartar or the like from the surface, especially dental and gum surfaces, in a manner similar to which an eraser rubs pencil markings off a page.

In a particularly preferred embodiment of the invention, the dental treatment composition comprises a toothpaste or a toothgel. Typically, the particulate erasing agent comprises between 20% and 40% of the toothpaste or toothgel composition. In an alternative embodiment, the dental treatment composition comprises particulate erasing agent in a powder form, along with instructions explaining how the composition is administered to the teeth.

The invention also relates to the use of a particulate erasing agent in a dental treatment composition, wherein the particles of the erasing agent are dimensioned to roll along a surface.

The invention also relates to a method of treating teeth comprising the steps of:
applying a suitable amount of a dental treatment composition according to the invention onto a suitable applicator for the composition;
using the applicator to rub the composition onto a surface of the teeth such that at least some of the particles of the erasing agent roll along at least a portion of the teeth; and
optionally rinsing the composition off the teeth.

Typically, the applicator is a toothbrush, interdental brush, or soft rubber cup. When the applicator is a brush, it may be manually, mechanically or electrically operated.

The invention also relates to the use of the process of the invention in one or more dental applications selected from the group comprising: teeth brushing; teeth whitening; teeth cleaning; plaque and tartar removal; and general cleaning or polishing of the teeth. In this specification, the term teeth should be taken to include gums and mucous membranes of the buccal cavity, and prosthetic parts such as crowns, bridges and complete or partial dentures. As such, the process may involve either blast application using some form of particle accelerator, or manual application, of the treating agent. Manual application includes conventional brushing, rubbing, polishing or the like.

The invention also relates to the use of the process of the invention in treating bone or in skin exfoliation treatment.

In another embodiment, the treatment composition is a household care treatment composition. Thus, for example, the treatment composition may be a hard surface cleaner which may take the form of a particulate solid, a gel or a fluid such as a cream. In one embodiment, the hard surface treatment composition is suitable for use in cleaning surfaces such as baths, showers, sinks, tiled surfaces and the like. In another embodiment, the hard surface treatment composition is suitable for cleaning kitchen utensils such as pots, pans and other cooking and eating utensils. In another embodiment, the hard surface treatment composition is suitable for cleaning and/or polishing brassware, silverware and other metallic objects.

The invention also relates to a method of treating a hard surface comprising the steps of:
applying a suitable amount of a hard surface treatment composition according to the invention onto a suitable applicator for the composition; using the applicator to rub the composition onto a hard surface such that at least some of the particles of the erasing agent roll along at least a portion of the hard surface; and
optionally rinsing the composition off the hard surface.

The invention also relates to a method of exfoliating skin comprising the steps of:
applying a suitable amount of an exfoliating treatment composition according to the invention onto a suitable applicator for the composition; using the applicator to rub the composition onto skin such that at least some of the particles of the erasing agent roll along at least a portion of the skin; and optionally rinsing the composition off the skin.

In one preferred embodiment, exfoliating treatment composition is applied by hand and in such cases the applicator may be a users hand. Otherwise, a particle accelerator may be used to apply the composition.

The invention also relates to the use of precipitated or aggregated alkali metal carbonate as an erasing agent in personal and household care treatment compositions, especially personal and household care cleaning compositions.

The invention also relates to the use of precipitated or aggregated alkali metal carbonate in dental treatments, personal washing, skin exfoliating, and household cleaning, compositions.

Typically, the precipitated or aggregated alkali metal carbonate is precipitated or aggregated calcium carbonate (PCC). Typically, the PCC has an average particle size between 30 and 1000 microns. Preferably, the PCC has an average particle size between 30 and 500 microns, more preferably between 30 and 100 microns. Typically, the PCC has an average particle size between 70 and 90 microns. Suitably, the PCC has an average particle size which is preferably more than 50 microns, particularly when it is used for dental treatment. Methods of sizing the particles will be well known to those skilled in the art. For example, vibrating sieves may be employed to separate out particles within a given range, for example, 70 to 90 microns.

In one embodiment of the invention, the dental treatment composition comprises at least 3% water (W/W), generally at least 5% water (W/W).

Preferably, the particles of the erasing agent comprise a precipitate or aggregate of an insoluble alkali metal salt. Typically, the salt is a carbonate. Suitably, the alkali earth metal is calcium. Most preferably, the particles of the erasing agent comprise a precipitate or aggregate of insoluble calcium carbonate. Typically, the precipitate or aggregate of insoluble calcium carbonate is obtained by a nitric acid method or a calcium oxide method. In one preferred embodiment, the particles of the erasing agent comprise an aggregate of calcite crystals formed into a round shape during crystallisation.

Preferably, the particles are generally round. In this specification the term "generally round" as applied to particles should be understood to mean any shape which of particle which enables the particle to easily assume a rolling motion when moved along a surface. As such, while the term is primarily intended to refer to spherical particles, in one aspect it is not intended to exclude other types of spheroids such as spheres having an oblong or elliptical shape. Ideally, the particles are round. Typically, the particles will have an irregular surface configuration.

Ideally, the particles are relatively soft. Generally, the particles have an average hardness of less than 10 Mohs, typically less than 8 Mohs, and preferably less than 6 Mohs. Typically, the particles will have an average hardness of at least 1 Mohs, and preferably of at least 2 Mohs. In a preferred embodiment of the invention, the particles will have an average hardness of about 3 Mohs. Typically, the particles have an average maximum diameter of between 30 and 1000 microns. Suitable methods of measuring Mohs hardness will be well known to those skilled in the field.

In one embodiment of the invention, the particles have an average maximum diameter of between 30 and 1000 microns, preferably between 60 and 120 microns, and most preferably between 70 and 80 microns.

Typically, the particulate erasing agent comprises between 1 and 75% of the total composition (W/W). Preferably, the particulate erasing agent comprises between 20 and 40%, most preferably between 25 and 35%, of the total composition (W/W).

In one embodiment of the invention, the dental treatment composition comprises a paste or a gel. Preferably, the dental treatment composition is a toothpaste. Alternatively, the dental treatment composition may comprise a teeth whitening composition, a plaque removal composition, a toothgel, a polishing paste, or the like.

In one embodiment of the invention, the dental treatment composition comprises a powder which, optionally, is used as an additive in a further component or components.

The invention also relates to the combination of a dental treatment composition according to the invention contained within a dispenser for the composition. Typically, the dispenser comprises a deformable tube. Other types of dental care composition dispensers are also envisaged such as, for example, piston pumps.

The invention also relates to a use of a particulate erasing agent in a dental treatment composition, wherein the particulate erasing agent comprises particles which are dimensioned to roll along a surface and which ideally have an average maximum diameter of between 30 and 1000 microns.

The invention also relates to the use of an alkali metal carbonate, typically precipitated or aggregated alkali metal carbonate, as a liquid hydrocarbon absorbing agent.

The invention also relates to a process for absorbing liquid hydrocarbon comprising the steps of bringing an alkali metal carbonate into contact with the liquid hydrocarbon, allowing the alkali metal carbonate absorb the liquid hydrocarbon, and removing the alkali metal carbonate.

In this specification, the term "liquid hydrocarbon" should be understood as including oil, petroleum and diesel.

Suitably, the process and use is suitable for cleaning up spilled oil.

### Brief Description of the Drawings

The invention will be more clearly understood from the following description of some embodiments thereof, given by way of example only, with reference to the following figures in which:
Fig 1 is an illustration of a particle of a treating agent according to the invention; and
Fig 2 illustrates the process of the invention.

### Detailed Description

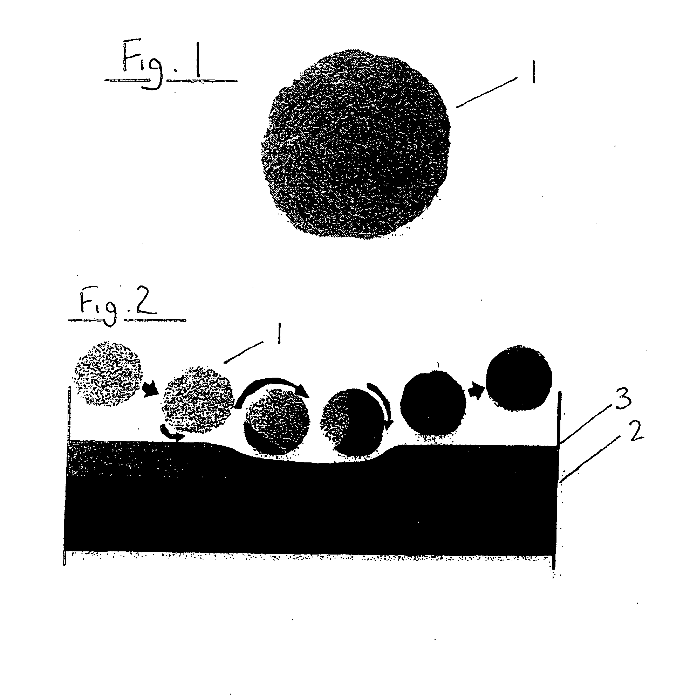

Referring to the drawings, and initially to Fig 1, there is illustrated a particle, indicated generally by the reference numeral 1, which is used in the process of the invention. The particle is a particle of precipitated calcium carbonate and has a generally round, and slightly irregular, shape and a rough, irregular, surface configuration.

Referring to Fig 2, the process of the invention is illustrated in which the particle 1 is rubbed along a surface 2 of a tooth having a coating 3 of plaque to be removed. Due to the nature and the round shape of the particle 1, upon impact the particle 1 rolls along the surface, rubbing the surface and absorbing the coating 3 onto a surface of the particle. This has the net effect of removing the coating from the surface without causing any damage to the surface.

### Example 1

### Method of production of particulate erasing agent (Calcium Oxide Method)

Production of insoluble calcium carbonate particles is carried out by providing free Ca⁺⁺ in a liquid with a PH over 7 by dissolving calcium oxide in water.

Addition of CO₂ results in the precipitation CaCO₃.

Ca⁺⁺ + 2OH⁻ + CO₂ → CaCO₃ + H₂O

Various other methods of production of particles forming part of treating agents according to the invention have been investigated using various types of substrates including plastic, metal and polymer.' Examples of these methods include:

### Chemical

There are numerous chemical methods for producing particulate erasing agents. Generally, chemical methods result in very fine powder particle sizes. Such methods include Sol Gel, chemical precipitation, Reaction, reduction (hydrogen in an autoclave to reduce metal salts to the metal), decomposition (eg metal carbonyls) and Electrolysis.

### Example 2

One specific method includes the steps of dissolving apatite in nitric acid (Nitric Acid Method). The thus formed liquid is cooled to crystallise out calcium nitrate. Calcium nitrate crystals are then separated from the thus-formed slurry by centrifugation or filtration. NH3 and CO2 is then added to the calcium nitrate, resulting in precipitation of CaCO3 and ammonium nitrate liquid. The precipitated CaCO3 is then separated by filtering.

### Spray drying

This is the most widely used industrial process involving particle formation and drying. It is highly suited for the continuous production of dry solids in either powder, granulate or agglomerate form from liquid feedstocks as solutions, emulsions and pumpable suspensions.

### Aggregation

The most common method of aggregation is where the constituents are physically mixed together with an organic binder. The solvent is then driven off and the resultant material sized. The binder should be burnt off during spraying. This process is used in the manufacture of NiAl, AlSi or polyester powders. The most common method of agglomeration is where the constituents are physically mixed together with an organic binder. The solvent is then driven off and the resultant material sized. The binder should be burnt off during spraying. This process is used in the manufacture of NiAl, AlSi-polyester powders.

The use of spray drying has become another common method for the aggregation of powders. Here, a slurry is formed with the constituents and this is then fed into a rotary spray head. Here, the slurry forms an atomised cloud which is solidified by an opposing warm air stream to produce a powder. This method is used for ceramics such as zirconia and cermets such as WC-cobalt. The powder is largely spherical but in the as spray dried state can be porous and friable. The material is often densified and stabilised by sintering and/or spray densification.

There are also methods of mechanical aggregation (eg the Hosakawa method) where for example a hard constituent is mechanically driven into a softer matrix particle to form a composite powder. Indeed, simple ball grinding can be used to mechanically alloy two or more constituents together.

Although sintering can be used as part of the spray drying process it can also be used alone as a method to manufacture powders. The constituents are mixed together and heated to get some solid state diffusion going and then the resultant product is crushed. A number of repeated cycles can be used to promote further alloying in which case the powder is called a "reacted" powder.

### Atomisation

There are a number of atomisation techniques which all rely on the production of a molten pool as the source. Atomisation methods include Rotating Electrode, Vibrating Electrode (arc), Centrifugal (from a melt) and Rapid Solidification (eg aluminium ribbon). However, by far the most commonly used methods are either water or gas atomisation.

### Others

- Solid State Reduction
- Electrolysis
- Electrodeposition

### Mechanical Comminution

The sources of commercially available precipitated calcium carbonate, and one means of manufacture, are listed in the paper entitled "Fine-Ground and Precipitated Calcium Carbonate" by Larisa Gorbaty, Andreas Leder and Yuka Yoshida, published in the Chemical Economics Handbook (1996 - SRI International).

### Toothpaste Compositions

As described above, the dental treatment composition of the invention may take the form of a toothpaste. In this regard, particulate erasing agent (precipitated calcium carbonate as formed in Example 2) may be added to a toothpaste composition in an amount of 30 % of the toothpaste composition (w/w). Prior to addition of the erasing agent it is sized using vibrating sieves to ensure that the particles have an average diameter of about 70 microns. Other suitable sizing methods will be apparent to those skilled in the art. Details of toothpaste formulations will be well known to those skilled in the field dental treatment compositions and will not be described in any detail in this specification.

### Personal Wash Compositions

The particulate erasing agent as produced in Example 2 (precipitated calcium carbonate) may be used in the formulation of personal wash compositions such as, for example, soap, shower gel, body wash, and the like. The amount of particulate erasing agent added to the compositions can be varied depending on the type of product. Otherwise, the composition of such personal wash composition will be known to those skilled in the field of personal wash formulation. Personal wash composition according to the invention are particularly suitable for washing oil and hydrocarbon-based soil from the skin and from other objects.

### Skin Exfoliating Compositions

The particulate erasing agent as produced in Example 2 (precipitated calcium carbonate) may be used in the exfoliation of skin in compositions such as, for example, soap, shower gel, body wash, and the like. The amount of particulate erasing agent added to the compositions can be varied depending on the type of product. Otherwise, the composition of such skin exfoliating compositions will be known to those skilled in the field of personal wash formulation objects.

### Household Care Composition

The formulation of household care composition, including hard surface cleaners in the forms of creams and particulate solids, will be well known to those skilled in the field of household cleaning and polishing composition formulation.

### Liquid Hydrocarbon Absorbing

Precipitated calcium carbonate (PCC) having a particle size of about 70 microns (as prepared above) is used to remove oil spilled on the ground. The PCC is poured onto the oil in an amount sufficient to cover the oil. The PCC is then left to absorb the oil. After a suitable amount of time, the PCC is then swept up thereby removing the oil.

The invention is not limited to the embodiments hereinbefore described which may be varied in both construction and process step without departing from the invention.

## Claims

1. Use of a particulate erasing agent in a personal washing composition, the particulate erasing agent being of the type comprising a plurality of particles, the particles comprising a precipitate or agglomerate of an insoluble alkali metal carbonate, being generally round and having an irregular surface configuration, being dimensioned to roll along a surface, and having an average maximum diameter of between 30 and 1000 microns.

2. Use as claimed in Claim 1, in which the particulate erasing agent is a precipitated alkali metal carbonate.

3. Use as claimed in Claim 2 in which the precipitated alkali metal carbonate is precipitated calcium carbonate.

4. Use as claimed in any of Claims 1 to 3, wherein the personal washing composition is selected from the group comprising: soap; shower gel; and body wash.

5. A personal washing composition comprising a particulate erasing agent of the type comprising a plurality of particles, the particles comprising a precipitate or agglomerate of an insoluble alkali metal carbonate, being generally round and having an irregular surface , configuration, being dimensioned to roll along a surface, and having an average maximum diameter of between 30 and 1000 microns.

6. A personal washing composition as claimed in Claim 5 in which the particulate erasing agent is a precipitated alkali metal carbonate.

7. A personal washing composition as claimed in Claim 6 in which the precipitated alkali metal carbonate is a precipitated calcium carbonate.

8. A personal washing composition as claimed in any of Claims 5 to 7, and selected from the group comprising: soap; shower gel; and body wash.
